# EUROPEAN PATENT SPECIFICATION

(11) **EP 1 496 757 B1**
(45) Date of publication and mention of the grant of the patent: **03.09.2008**
(21) Application number: 03728439.5
(22) Date of filing: 18.04.2003
(51) Int. Cl.: A23L 1/30, A61K 31/201, A61K 31/20, A23L 1/308

(54) **COMPOSITIONS COMPRISING SHORT AND LONG CHAIN FATTY ACIDS AND METHODS OF THEIR USE FOR THE MANAGEMENT OF BODY WEIGHT**
ZUSAMMENSETZUNGEN, DIE KURZ-UND LANGKETTIGE FETTSÄUREN ENTHALTEN, SOWIE VERFAHREN ZU IHRER ANWENDUNG FÜR DIE KONTROLLE DES KÖRPERGEWICHTS
COMPOSITIONS COMPRENANT DES ACIDES GRAS A CHAINE COURTE ET A CHAINE LONGUE ET METHODES D'UTILISATION DANS LA GESTION DU POIDS CORPOREL

(30) Priority: 24.04.2002 US 376032 P; 24.04.2002 US 376060 P; 24.04.2002 US 375653 P; 07.08.2002 US 214009
(43) Date of publication of application: 19.01.2005
(73) Proprietor: THE PROCTER & GAMBLE COMPANY, Cincinnati, Ohio 45202 (US)
(72) Inventor: KELM, Gary, Robert, Cincinnati, OH 45247 (US); CLYMER, Jeffrey, W., Mason, OH 45040 (US); BHARAJ, Satinder, Singh, Hamilton, OH 45011 (US); STARCHER, Mary, Ann, Hamilton, OH 45011 (US); FRANCIS, Cynthia, Elodi, Cincinnati, OH 45249 (US)
(74) Representative: Clemo, Nicholas Graham
(86) International application number: PCT/US2003/012047
(87) International publication number: WO 2003/090557

(56) References cited:
- WO-A-02/00042
- GB-A- 2 353 471
- US-A- 5 662 953
- US-A- 5 700 782

## Description

### FIELD OF THE INVENTION

The present invention relates to compositions that are useful for the management of body weight. In particular, the present compositions may be of a variety of forms, including foods, beverages, tablets, capsules, emulsions and other orally administrable forms. The compositions are useful for the treatment of mammals, for example humans and companion animals.

### BACKGROUND OF THE INVENTION

The incidence of obesity in the general population of the United States has dramatically increased over the last decade, with over 50% of the population considered overweight or obese. A similar trend is observed in other countries as the so-called "Western Diet" is adopted. Since obesity is associated with a variety of co-morbidities such as diabetes, hypertension, and atherosclerosis, this increase is a major health concern.

A number of approaches have been proposed or used to help mammals reduce energy intake (*e.g.,* caloric intake) with the objective of managing body weight. These approaches include use of agents to act on the central nervous system to increase levels of serotonin, and those acting in the gastrointestinal tract to reduce digestion and/or absorption of nutrients. These approaches suffer from potential side effects that reduce their utility for long-term body weight management.

A different approach involves the introduction of nutrients directly into the distal small intestine in an attempt to reduce concomitant food intake. This approach uses natural materials and is believed to function *via* interactions of the nutrients with putative receptors throughout the small intestine, particularly in the distal small intestine (jejunum, ileum), that are believed to participate in the natural mechanisms that induce termination of a meal. This offers the potential advantage of a reduction of side effects due to the use of natural materials and mechanism. Reported data in animals and humans are based on using catheters and naso-gastric tubes respectively, to directly introduce nutrients into the small intestine. Many of the reported studies have employed lipids. For example, infusion of a corn oil emulsion into the small intestine (jejunum or ileum) reduces food intake at a concomitant meal such that total caloric intake (meal plus infusate) is significantly or directionally reduced. See Welch et al., "Effect of Ileal and Intravenous Infusions of Fat Emulsions on Feeding and Satiety in Human Volunteers," Gastroenterology, Vol. 89, pp. 1293-1297 (1985) and Welch et al., "Comparisons of the Effects on Satiety and Eating Behavior of Infusion of Lipid into the Different Regions of the Small Intestine," Gut, Vol. 29, pp. 306-311 (1988). Intravenous administration of a triacylglycerol emulsion failed to reduce total caloric intake in several studies. See Walls and Koopmans, "Effect of Intravenous Nutrient Infusions on Food Intake in Rats," Physiology & Behavior, Vol. 45, pp. 1223-1226 (1989).

A variety of animal studies have been reported wherein nutrients are infused into the small intestine to reduce food intake. Meyer *et al.* have conducted a series of studies investigating the effects of infused nutrients upon total caloric intake in rats constrained to three hours of feeding. They found that emulsions of fatty acids greater than 10 carbons in length, solutions of monomeric carbohydrates only having affinity for the glucose transporter, and solutions of the amino acids tryptophan and phenylalanine suppressed total caloric intake when infused in the duodenum or mid-gut (jejunum), or into the colon. These investigators also demonstrated that pre-meals of triacylglycerol failed to suppress total caloric intake in rats constrained to seven hours of feeding. See Meyer et al., "Chemical Specificities and Intestinal Distributions of Nutrient-Driven Satiety," Am. Journal Physiology, Vol. 275, R1293-R1307 (1998).

More recently, Cox *et al.* have shown that jejunally infused neat linoleic acid or oleic acid (0.2 mL/hr for seven hours) will significantly reduce total daily caloric intake in rats, whereas a long chain triacylglycerol (corn oil) will not. These authors also demonstrated that the reduction is maintained over 20 days of dosing, resulting in a significant difference in weight between treated and control animals. See Cox et al., "Suppression of Food Intake, Body Weight, and Body Fat by Jejunal Fatty Acid Infusions," Am. Journal Physiology, Regulatory Integrative Comparative Physiology, Vol. 278, R604-R610 (2000).

Long chain fatty acid compositions designed to release such fatty acid in the stomach have most recently been described. See WO 02/00042.

Others have described the effects of shorter chain components such as sodium propionate in food forms such as bread. For example, Liljeberg and Björck have described induction of satiety in humans, which was attributed to a delayed gastric emptying rate, through the inclusion of sodium propionate in bread. However, sodium propionate was found to have no effect on the gastric emptying in rats. See Liljeberg and Björck, "Delayed Gastric Emptying Rate as a Potential Mechanism for Lowered Glycemia After Eating Sourdough Bread: Studies in Humans and Rats Using Test Products with Added Organic Acids or an Organic Salt," Am. J. Clin. Nutr., Vol. 64, pp. 886 - 893 (1996). With respect to other reported benefits of short chain fatty acids see also, Todesco et al., "Propionate Lowers Blood Glucose and Alters Lipid Metabolism in Healthy Subjects," Am. J. Clin. Nutr., Vol. 54, pp. 860 - 865 (1991); Wright et al., "Propionate Inhibits Hepatocyte Lipid Synthesis," Society for Experimental Biology and Medicine, Vol. 195, pp. 26 - 29 (1990); Berggren et al., "Influence of Orally and Rectally Administered Propionate on Cholesterol and Glucose Metabolism in Obese Rats," British Journal of Nutrition, Vol. 76, pp. 287 - 294 (1996); Cameron-Smith et al., "Effect of Propionate on In Vivo Carbohydrate Metabolism in Streptozocin-Induced Diabetic Rats," Metabolism, Vol. 43, No. 6, pp. 728 - 734 (1994); Venter et al., "Effects of Dietary Propionate on Carbohydrate and Lipid Metabolism in Healthy Volunteers," The American Journal of Gastroenterology, Vol. 85, No. 5, pp. 549 - 553 (1990); Farningham and Whyte, "The Role of Propionate and Acetate in the Control of Food Intake in Sheep," Vol. 70, pp. 37 - 46 (1993); Farningham et al., "Satiety Signals in Sheep: Involvement of CCK, Propionate, and Vagal CCK Binding Sites, Physiology and Behavior, Vol. 54, pp. 437 - 442 (1993). US 5,635,535 describes the use of propionic acid or its salts, esp. calcium propionate, for the reduction of body weight; WO 99/11254 describes the use of short chain fatty acid and salts, esp. calcium acetate, for the treatment of obesity and US 6,077,556 discloses solid sucrose polyesters comprising both short and long chain fatty acid groups which are useful for calory reduction.

Accordingly, the use of certain short and long chain fatty acid compositions have been separately described *via* various administration routes for the induction of satiety and other body weight management effects. It is clear that the use of such compositions is of emerging and unique importance, and it would therefore be beneficial to advance studies in this area.

The present inventors have unexpectedly discovered that combinations of certain long chain fatty acid components (LCFA), together with certain short chain fatty acid components (SCFA), are effective to provide true synergistic benefits in the field of body weight management. In particular, these combinations are effective in a greater than additive manner, which is not expected in the art. The present inventors have therefore discovered an important advance in the efficacy of body weight management methods, by providing uniquely efficacious compositions not described previously in the art.

### SUMMARY OF THE INVENTION

The present invention relates to compositions comprising particular short and long chain fatty acid components (SCFAs and LFCAs), suitable for oral administration, wherein the compositions are useful for the management of body weight. For example, body weight management may be effected *via* induction of satiety by a composition of the invention. In particular, the present compositions comprise:
a) from 0.001% to 8% of a short chain fatty acid component selected from acetic acid, propionic acid and butyric acid; methyl, ethyl, n-propyl, iso-propyl, n-butyl and isobutyl esters thereof; salts thereof; and mixtures thereof; and
b) from 0.001% to 10% of a long chain fatty acid component selected from C₁₀ - C₂₄ fatty acids, non-glyceryl esters of C₁₀ - C₂₄ fatty acids, and mixtures thereof.

The invention further relates to the use of the SCFAs and LFCAs in the manufacture of a medicament for managing the body weight of a mammal.

### DETAILED DESCRIPTION OF THE INVENTION

All percentages and ratios are calculated by weight unless otherwise indicated. All percentages and ratios are calculated based on the total composition unless otherwise indicated.
Referenced herein are trade names for components including various ingredients utilized in the present invention. The inventor herein does not intend to be limited by materials under a certain trade name. Equivalent materials (*e.g*., those obtained from a different source under a different name or reference number) to those referenced by trade name may be substituted and utilized in the descriptions herein.
In the description of the invention various embodiments or individual features are disclosed. As will be apparent to the ordinarily skilled practitioner, all combinations of such embodiments and features are possible and can result in preferred executions of the present invention.
The compositions herein may comprise, consist essentially of, or consist of any of the elements as described herein.

### Definitions

As used herein, "companion animal" means a domestic animal. Preferably, "companion animal" means a domestic dog, cat, rabbit, ferret, horse, cow, or the like. Most preferably, "companion animal" means a domestic dog or cat.
As used wherein, the term "safe and effective" means effective for the management of body weight in a mammal (preferably a human or companion animal) without undue adverse side effects (such as toxicity, irritiation, or allergic response), commensurate with a reasonable benefit/risk ratio when used in the manner of this invention.

### Compositions of the Present Invention

The present compositions are useful for a variety of purposes, particularly in the management of body weight. In particular, without intending to be limited by theory, it is believed that the present compositions are useful for inducing a satiety response, as well as other biological responses which are important in the modulation of body weight. The present inventors have unexpectedly discovered that combinations of certain long chain fatty acid components (LCFA), together with certain short chain fatty acid components (SCFA), are effective to provide true synergistic benefits in the field of body weight management, which is not expected in the art.

The present inventors have discovered that the combination of the SCFA and LCFA, as defined herein, results in an unexpected, true synergistic response that has been found to be an important advance in the modulation of body weight. In particular, the inventors have discovered that the combination of SCFA and LCFA, again as specifically defined herein, results in a true synergistic response which is greater than would have been expected. For example, the inventors have found that wherein non-optimized levels of SCFA and LCFA are dosed separately, to separate dosing groups, the reduction in caloric intake amongst both dosing groups is also found to be non-optimized. In contrast, for example, where the SCPA and LCFA are dosed together, in the same dousing group, using the same non-optimized levels of each, an effective, synergistic reduction of caloric intake is achieved. Accordingly, the inventors have discovered an important combination of components that is particularly useful for the advancement of effective body weight management compositions.
The various components of the present compositions are described further as follows:

### The Short Chain Fatty Acid Component

The short chain fatty acid component (herein often referenced as "SCFA"), is selected from acetic acid, propionic acid, and butyric acid, Methyl, ethyl, A-propyl, iso-proply, n-butyl and iso-butyl esters thereof, salts thereof; and mixtures thereof. These SCFAs are commonly understood in the art.

Wherein the SCFA is acetic acid, propionic acid, or butyric acid, it is preferred to utilize propionic acid. It is also preferred that the SCFA is selected from propionic acid, salts of propionic acid, Methyl, ethyl, A-propyl, iso-proply, n-butyl and iso-butyl esters of propionic acid, and mixtures thereof.
Among the group of acetic acid, propionic acid, and butyric acid, Methyl, ethyl, A-propyl, iso-proply, n-butyl and iso-butyl esters thereof, salts thereof, and mixtures thereof, the salts of acetic acid, propionic acid, or butyric acid ("salts thereof) are most particularly preferred. The salts of acetic acid, propionic acid, or butyric acid may be any safe and effective salt of such acid. To illustrate, certain preferred salts may include calcium salts, sodium salts, magnesium salts, and potassium salts. Among these calcium and sodium salts of the acetic, propionic, or butyric acid may be particularly preferred, with calcium salts being most particularly preferred.
For example, preferred salts of propionic acid include calcium propionate, sodium propionate, magnesium propionate, potassium propionate, and mixtures thereof. Among these, calcium propionate and sodium propionate may be particularly preferred, with calcium propionate being among the most preferred.
As an additional illustration, amino acid salts may be utilized. For example, a carnitine or lysine salt of the acetic, propionic, or butyric acid may be utilized. The ordinarily skilled artisan will recognize that various other amino acids may be utilized as well.
As an additional benefit to the body weight management effects that the salts of propionic acid confer herein, certain salts may also beneficially provide a nutritive, or other, benefit. For example, the use of calcium propionate may additionally provide a source of calcium, which is known to be useful for a variety of health benefits.
Where the SCFA is an ester of propionic acid, the component is depicted as follows: 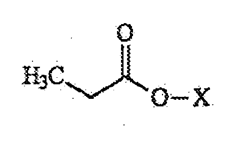 where X is the ester chain or the ester of propionic acid, and is selected from methyl esters (*i.e*., X is -CH₃), ethyl esters, *n*-propyl esters, *iso*-propyl esters, *n*-butyl esters, *iso*-butyl esters, and mixtures thereof. To illustrate, methyl propionate, ethyl propionate, *n*-propyl propionate, *iso*-propyl propionate, *n*-butyl propionate, *iso*-butyl propionate are examples of esters of propionic acid that may be used herein. Such esters of acetic or butyric acid may be selected as well.

The level of SCFA contained within a given composition or medicament will typically be dependent upon the particular dosage form selected for use, particularly wherein such level is expressed as a weight percentage relative to all components present within the composition. For example, as further described herein, particularly preferred dose forms will include tablets, capsules, other concentrated orally deliverable forms, foods, beverages, and the like. Typically, a tablet or capsule may comprise a higher level of SCFA, by weight percent of the composition, relative to (for example) a beverage.
For example, certain dosage forms of the present medicaments may optionally comprise from 0.0001% to 50% of the SCFA, by weight . In another embodiment, the compositions comprise from 0.001% to 8%, more preferably from 0.01% to 4% of the SCFA, all by weight of the composition. In yet a further embodiment, the compositions may comprise from 0.01% to 2% of the SCFA, by weight of the composition.
For example, tablet or capsule forms of the present invention (or other like forms) may optionally comprise from 1% to 50% of the SCFA, by weight. As a further example, emulsion forms may optionally comprise from 0.1% to 10% of the SCFA, more preferably from 0.5% to 5% of the SCFA, by weight . Additionally, food or beverage forms may often comprise from 0.0001% to 8% of the SCFA, by weight of the composition.

A single dose of the composition of the present invention may optionally comprise from about 0.01 grams to about 10 grams of SCFA, more preferably from about 0.04 grams to about 1 grams, all per kilogram body weight of the subject to which the composition is administered. As used herein, the single dose refers to the amount of composition that is typically consumed at a given time.

### The Long Chain Fatty Acid Component

The long chain fatty acid component (herein often referenced as "LCFA"), is selected from C₁₀-C₂₄ fatty acids, non-glyceryl esters of C₁₀-C₂₄ fatty acids, and mixtures thereof. As used herein, the LCFA contains a fatty acid chain, or wherein the fatty acid material is a fatty acid ester, contains a fatty acid chain and an ester chain. Thus, wherein the LCFA is a fatty acid, the material is depicted as follows:

R-COOH

wherein "R" is the fatty acid chain which is a saturated or unsaturated chain having from 10 to 24 carbon atoms, and wherein "COOH" is a carboxylic acid moiety. More preferably. "R" is a saturated or unsaturated chain having from 12 to 24, most preferably from 16 to 18 carbon atoms. Also preferably, the fatty acid chain contains from 0 to 3 double bonds. Most preferably, the fatty acid chain is unsaturated, in particular having one or two double bonds.

Wherein the fatty acid material is a non-glyceryl ester of a fatty acid (*i.e*., a "non-glyceryl ester thereof"), the material is depicted as follows:

R-COOR'

Wherein R is the fatty acid chain as defined above, and R' is the ester chain, with the carboxylate moiety "COO" linking the two together. The ester chain is a straight or branched chain of carbon atoms that is hydrolyzable in the presence of mammalian digestive enzymes, preferably human digestive enzymes, and typically contains 8 carbon atoms or less. The ester chain more preferably contains from 1 to 5 carbon atoms and, again, may be a straight (for example, *n-*propyl) or branched (for example, *iso*-propyl) chain. Highly preferred ester chains include those that form methyl, esters (*i.e*., R' is -CH₃), ethyl esters, *n*-propyl esters, *iso*-propyl esters, *n*-butyl esters, *iso*-butyl esters, and mixtures thereof. These ester chains that form ethyl esters are particularly preferred.

In a preferred embodiment of the present invention, the LCFA is selected from lauric acid, lauroleic acid, myristic acid, myristoleic acid, pentadecanoic acid, palmitic acid, palmitoleic acid, margaric acid, stearic acid, dihydroxystearic acid, oleic acid, ricinoleic acid, elaidic acid, linoleic acid, alpha-linolenic acid, dihomogamma-linolenic acid, eleostearic acid, licanic acid, arachidonic acid, arachidic acid, eicosenoic acid, eicosapentaenoic acid, behenic acid, erucic acid, docosahexaenoic acid, lignoceric acid, non-glyceryl esters thereof, and mixtures thereof.

In a particularly preferred embodiment of the present invention, the fatty acid material is selected from lauric acid, lauroleic acid, myristic acid, myristoleic acid, pentadecanoic acid, palmitic acid, palmitoleic acid, margaric acid, stearic acid, dihydroxystearic acid, oleic acid, ricinoleic acid, elaidic acid, linoleic acid, alpha-linolenic acid, dihomeogamma-linolenic acid, eleostearic acid, licanic acid, arachidonic acid, arachidic acid, eicosenoic acid, behenic acid, erucic acid, lignoceric acid, non-glyceryl esters thereof, and mixtures thereof. Additionally, particularly preferred fatty acid materials include oleic acid, linoleic acid, esters thereof, and mixtures thereof. Preferred non-glyceryl esters of this embodiment include ethyl oleate, ethyl linoleate, and mixtures thereof. As an example, ethyl oleate may be obtained from a variety of sources, including Victorian Chemical Co., Richmond, Victoria Australia; Penta Manufacturing Co., Livingston, NJ; and Croda, Inc., Parsippany, NJ.

Like the SCFA, the level of LCFA contained within a given composition or medicament will typically be dependent upon the particular dosage form selected for use, particularly wherein such level is expressed as a weight percentage relative to all components present within the composition. For example, as further described herein, particularly preferred dose forms will include tablets, capsules, other concentrated orally deliverable forms, foods, beverages, and the like. Typically, a tablet or capsule may comprise a higher level of LCFA, by weight percent of the composition, relative to (for example) a beverage.
For example, certain dosage forms of the present medicaments may optionally comprise from 0.0001% to 90% of the LCFA, by weight. In another embodiment, the compositions comprise from 0.001% to 10%, more preferably from 0.01% to 5% of the LCFA, all by weight of the composition. In yet a further embodiment, the compositions may comprise from 0.01% to 3% of the LCFA, by weigh of the composition.
For example, tablet or capsule forms of the present invention (or other like forms) may optionally comprise from 1% to 90% of the LCFA, by weight. As a further example, emulsion forms may optionally comprise from 10% to 40% of the LCFA, more preferably from 15% to 30% of the LCFA, by weight. Additionally, food or beverage forms may often comprise from 0.0001% to 10% of the LCFA, by weight of the composition.

A single dose of the composition of the present invention may optionally comprise from about 0.001 grams to about 10 grams of LCFA, more preferably from about 0.004 grams to about 1 grams, all per kilogram body weight of the subject to which the composition is administered. As used herein, the single dose refers to the amount of composition that is typically consumed at a given time.

### Optional Ratios of LCFA to SCFA

In an optional embodiment of the present invention, the inventors have discovered that the body weight management efficacy of the compositions and medicaments described herein may be particularly optimized by adjusting the ratio of LCFA to SCFA, by weight (of the SCFA to the SCFA). For example, in one optional embodiment herein, the ratio of LCFA to SCFA is 40 : 1 or less, also optionally 20 : 1 or less, also optionally 10 : 1 or less, and also optionally 5 : 1 or less, all by weight. In a particular advantageous embodiment herein, the ratio of LCFA to, SCFA is 10 : 1 or less, by weight.

### Optional Components and Uses of the Present Compositions

The compositions, and medicaments herein are useful as, for example, pharmaceutical compositions, over-the-counter compositions, dietary supplements, medial foods, foods, and beverages, as well as a variety of other compositions.
The compositions of the present invention may comprise additional optional components to, for example, enhance their performance or to otherwise render the composition more suitable for use as an industrial or consumer product. Non-limiting examples of optional components are given below:

### Water

Water may be included in the compositions of the present invention, for example wherein the compositions are food or beverage compositions. As used herein, the term "water" includes the total amount of water present in the composition including water which is derived from, for example, milk protein, fruit juice, or vegetable juice, as well as any added water. Wherein water is utilized, the water is optionally included at, for example, levels from about 10% to about 99.999%, more preferably from about 5% to about 99%, still more preferably at least about 50%, even more preferably at least about 70%, and most preferably from about 70% to about 99%, by weight of the composition. Ready-to-drink beverage compositions will typically comprise at least 70% water, preferably from 75% to 99% water, all by weight of the composition.

### Carbohydrates

The compositions of the present invention may include any of a variety of carbohydrates. Often such carbohydrates may be included is compositions which are in food or beverage dosage form. For example, certain sweeteners are carbohydrates.

### Sweeteners

The compositions of the present invention may optionally contain an effective amount of one or more sweeteners, including carbohydrate sweeteners and natural or artifical no/low calorie sweeteners. The amount of the sweetener used in the compositions of the present invention typically depends upon the particular sweetener used and the sweetness intensity desired. For no/low calorie sweeteners, this amount varies depending upon the sweetness intensity of the particular sweetener.

The compositions of the present invention can be sweetened with any of the carbohydrate sweeteners, preferably monosaccharides and / or disaccharides. Sweetened compositions, particularly beverages, will typically comprise from about 0.1% to about 40%, more preferably from about 0.1% to about 20%, and most preferably from about 6% to about 14%, sweetener, all by weight of the composition. These sweeteners can be incorporated into the compositions in solid or liquid form (such as a syrup).

Preferred sugar sweeteners for use in compositions of the present invention are sucrose, fructose, glucose, and mixtures thereof. Fructose can be obtained or provided as liquid fructose, high fructose corn syrup, dry fructose or fructose syrup. Other naturally occurring sweeteners or their purified extracts, such as glycyrrhizin, the protein sweetener thaumatin, the juice of Luo Han Guo disclosed in, for example, U.S. Patent No. 5,433,965, and the like can also be used in the compositions of the present invention.

Suitable no/low calorie sweeteners include, for example, saccharin, cyclamates, L-aspartyl-L-phenylalanine lower alkyl ester sweeteners (*e*.*g*., aspartame); L-aspartyl-D-alanine amides disclosed in Brennan et al., U.S. Patent No. 4,411,925; L-aspartyl-D-serine amides disclosed in U.S. Patent 4,399,163; L-aspartyl-L-1-hydroxymethylalkaneamide sweeteners disclosed in U.S. Patent No. 4,338,346; L-aspartyl-1-hydroxyethyalkaneamide sweeteners disclosed in U.S. Patent No. 4,423,029; L-aspartyl-D-phenylglycine ester and amide sweeteners disclosed in European Patent Application 168,112; N-[N-3,3-dimethylbutyl)-L-alpha-aspartyl]-L-phenylalanine 1-methyl ester sweeteners disclosed in WO 99/30576, published June 24, 1999; alltame, thaumatin; dihydrochalcones; cyclamates; steviosides; glycyrrhizins, synthetic alkoxy aromatics, such as Dulcin and P-4000; sucralose; suosan; miraculin; monellin; sorbitol, xylitol; talin; cyclohexylsulfamates; substituted imidazolines; synthetic sulfamic acids such as acesulfame, acesulfame-K and n-substituted sulfamic acids; oximes such as perilartine; peptides such as aspartyl malonates and succanilic acids; dipeptides; amino acid based sweeteners such as gem-diaminoalkanes, meta-aminobenzoic acid, L-aminodicarboxylic acid alkanes, and amides of certain alpha-aminodicarboxylic acids and gem-diamines; and 3-hydroxy-4-alkyloxyphenyl aliphatic carboxylates or heterocyclic aromatic carboxylates; erythritol; and mixtures thereof.

### Coloring Agents

One or more coloring agents may optionally be utilized in the compositions of the present invention. For example, natural or artificial colors may be used.
FD&C dyes (*e*.*g*., yellow #5, blue #2, red # 40) and / or FD&C lakes are preferably used. By adding the lakes to the other powdered ingredients, all the particles, in particular the colored iron compound, are completely and uniformly colored and a uniformly colored composition is attained. Preferred lake dyes which may be used in the present invention are the FDA-approved Lake, such as Lake red #40, yellow #6, blue #1, and the like. Additionally, a mixture of FD&C dyes or a FD&C lake dye in combination with other conventional food and food colorants may be used.
Other coloring agents, for example, natural agents may be utilized.
The amount of coloring agent used will vary, depending on the agents used and the intensity desired in the finished composition. Generally, if utilized, the coloring agent is typically present at a level of from about 0.0001% to about 0.5%, preferably from about 0.001% to about 0.1%, and most preferably from about 0.004% to about 0.1%, by weight of the composition.

### Flavor Agents

The compositions herein may optionally comprise one or more flavor agents. Preferably, such flavor agents are included in the beverage compositions and are typically selected from fruit juice, fruit flavors, botanical flavors, and mixtures thereof. Wherein fruit juice is included, the beverages of the present invention can comprise from about 0.1% to about 99%, preferably from about 1% to about 50%, more preferably from about 2% to about 30%, and most preferably from about 5% to about 20%, fruit juice. As measured herein, the weight percentage of fruit juice is based on a single strength 2° to 16° Brix fruit juice. The fruit juice can be incorporated into the beverage as a puree, comminute, or as a single strength or concentrated juice. Especially preferred is incorporation of the fruit juice as a concentrate with a solids content (primarily as sugar solids) of from about 20° to about 80° Brix.

The fruit juice can be any citrus juice, non-citrus juice, or mixture thereof, which are known for use in dilute juice beverages. The juice can be derived from, for example, apple, cranberry, pear, peach, plum, apricot, nectarine, grape, cherry, currant, raspberry, gooseberry, elderberry, blackberry, blueberry, strawberry, lemon, lime, mandarin, orange, grapefruit, cupuacu, potato, tomato, lettuce, celery, spinach, cabbage, watercress, dandelion, rhubarb, carrot, beet, cucumber, pineapple, coconut, pomegranate, kiwi, mango, papaya, banana, watermelon, passion fruit, tangerine, and cantaloupe. Preferred juices are derived from apple, pear, lemon, lime, mandarin, grapefruit, cranberry, orange, strawberry, tangerine, grape, kiwi, pineapple, passion fruit, mango, guava, raspberry and cherry. Citrus juices, preferably grapefruit, orange, lemon, lime, and mandarin juices, as well as juices derived from mango, apple, passion fruit, and guava, as well as mixtures of these juices are most preferred.

Fruit flavors may also be utilized. As described above with respect to flavor emulsions, fruit flavors may be derived from natural sources such as essential oil and extracts, or can be synthetically prepared. Fruit flavors may be derived from fruits through processing, particularly concentrating.

Botanical flavors may also be utilized. As used herein, the term "botanical flavor" refers to a flavor derived from parts of a plant other than the fruit; *i*.*e*., derived from nuts, bark, roots, and / or leaves. Also included within the term "botanical flavor" are synthetically prepared flavors made to simulate botanical flavors derived from natural sources. Botanical flavors can be derived from natural sources such as essential oils and extracts, or can be synthetically prepared. Suitable botanical flavors include tea, coffee, chocolate, vanilla, jamaica, kola, marigold, chrysanthemum, chamomile, ginger, valerian, yohimbe, hops, eriodictyon, ginseng, bilberry, rice, red wine, mango, peony, lemon balm, nut gall, oak chip, lavender, walnut, gentiam, luo han guo, cinnamon, angelica, aloe, agrimony, yarrow and mixtures thereof. Particularly preferred flavors include chocolate or vanilla.

### Protein Components

The present compositions may optionally comprise a protein component, for example, animal or plant protein.
A non-limiting example of such protein includes milk protein. Milk protein comprises a protein selected from whey, casein, and mixtures thereof. The milk protein may be the protein itself, for example as sodium or calcium caseinate, or may be a component comprising the protein and one or more other materials. For example, various milk proteins are known to one of ordinary skill in the art and may be utilized as the protein component herein.

Wherein the milk protein is delivered as the protein itself, casein is the preferred protein for use herein. However, whey or mixtures of casein and whey may also be included. Casein may be utilized as a variety of forms including, for example, sodium or calcium caseinate. Mixtures of sodium and calcium caseinate are preferred for use herein.
Milk proteins include mammalian or vegetable milks such as, for example, whole milk, skim milk, condensed milk, dry milk powder, milk protein concentrate, milk protein isolate, milk protein hydrosylate, and mixtures thereof. To illustrate, milk protein concentrate is prepared via milk ultrafiltration or other means such that the lactose or salt content is reduced, thereby enhancing the protein content. In dry and condensed milk, water is removed but all other components of milk are substantially maintained. All forms of milk protein can comprise, for example, intact milk protein, milk protein hydrosylate, or any combination thereof.
Where used, the amount of protein in the present compositions will depend upon a variety of factors including, for example, whether the protein is the protein itself or delivered as a combination of components (for example, whole milk), the amount of protein desired in the final composition, and the like. In one optional embodiment, the compositions comprise at least about 0.5% protein, by weight of the composition. In another embodiment, the compositions comprises from about 0.5% to about 10% protein, even more preferably from about 0.5% to about 8% protein, and most preferably from about 0.5% to about 5% protein, all by weight of the composition.

### Nutrients

The compositions herein may be fortified with one or more nutrients, defined herein as one or more vitamins and / or minerals.
As an example, wherein a given mineral is present in the composition, the composition optionally comprises at least about 1%, preferably at least about 5%, more preferably from about 10% to about 200%, even more preferably from about 40% to about 150%, and most preferably from about 60% to about 125% of the USRDI of such mineral. Also an example, wherein a given vitamin is present in the composition, the composition optionally comprises at least about 1%, preferably at least about 5%, more preferably from about 10% to about 200%, even more preferably from about 20% to about 150%, and most preferably from about 25% to about 120% of the USRDI of such vitamin. The U.S. Recommended Daily Intake (USRDI) for vitamins and minerals are defined and set forth in the Recommended Daily Dietary Allowance-Food and Nutrition Board, National Academy of Sciences-National Research Council.
Non-limiting examples of vitamins include vitamins A, B₁, B₂, B₃, B₆ and B₁₂, folic acid, pantothenic acid, folic acid, C, D, and E.

Non-limiting examples of minerals include calcium, iodine, chromium, magnesium, manganese, molybdenum, selenium, phosphorous, magnesium, zinc, iodine, iron, and copper. As an example, any soluble salt of these minerals suitable for inclusion in edible compositions may optionally be used.

### Fiber

In a particularly preferred embodiment herein, the compositions comprise one or more fibers. Fibers are well-known in the art and include complex carbohydrates resistant to digestion by mammalian enzymes, such as the carbohydrates found in plant cell walls and seaweed, and those produced by microbial fermentation. Examples of these complex carbohydrates are oat fibers, brans, celluloses, hemicelluloses, pectins, gums and mucilages (e.g., guar gum and gum arabic), seaweed extract, carrageenan, and biosynthetic gums. Sources of the cellulosic fiber include vegetables, fruits, seeds, cereals, and man-made fibers (for example, by bacterial synthesis). Commercial fibers such as purified plant cellulose, or cellulose flour, can also be used. Naturally occurring fibers include fiber from whole citrus peel, citrus albedo, sugar beets, citrus pulp and vesicle solids, apples, apricots, and watermelon rinds.
Other preferred fibers for use herein include arabinogalactans. Non-limiting examples of preferred, commercially available sources of arabinogalactan include LAREX UF, LARACARE A200, IMMUNENHANCER (CAS No. 9036-66-2), CLEARTRAC, FIBERAID, and AC-9, all commercially available from (for example) Larex, Inc. of St. Paul, Minnesota.

These dietary fibers may be in a crude or purified form. The dietary fiber used may be of a single type (*e*.*g*., cellulose), a composite dietary fiber (*e*.*g*., citrus albedo fiber containing cellulose and pectin), or some combination of fibers (*e*.*g*., cellulose and a gum). The fibers can be processed by methods known to the art.

Wherein a fiber is utilized, the desired total level of fiber for the present compositions of the present invention is typically from about 0.001% to about 15%, preferably from about 0.01% to about 10%, more preferably from about 0.1% to about 10%, and most preferably from about 1% to about 9%, all by weight of the composition. The total amount of fiber includes any added fiber as well as any soluble dietary fiber naturally present in any other component of the present invention.

### pH

The compositions of the present invention may have various pH levels. The pH of a given composition may be particularly important wherein the composition is a beverage composition. For example, the compositions may be acidic in nature (for example a pH of from about 3 to about 5) or more basic. Preferred compositions of the present invention have a pH of from about 6 to about 8, more preferably from about 6.3 to about 7.4.
Organic as well as inorganic edible acids may be used to lower the pH of the beverage composition. The acids can be present in their undissociated form or, alternatively, as their respective salts, for example, potassium or sodium hydrogen phosphate, potassium or sodium dihydrogen phosphate salts. The preferred acids are edible organic acids including citric acid, malic acid, fumaric acid, adipic acid, phosphoric acid, gluconic acid, tartaric acid, ascorbic acid, acetic acid, phosphoric acid or mixtures thereof. The most preferred acids are citric and malic acids. Glucono Delta Lactone (GDL) is also a preferred acid for use herein, particularly wherein it is desired to reduce pH without introducing excessive acidic, or tart, flavor in to the final composition.

For those compositions that are more basic in nature, various bases may be utilized. For example, sodium hydroxide or potassium hydroxide may be used herein.

### Methods of the Present Invention

As used herein, the term "body weight management" means an effect selected from the group consisting of inducing satiety (*e*.*g*., controlling or curbing appetite), effecting the reduction of energy (*e.g*., caloric) intake, effecting weight loss, inhibiting weight gain, maintaining weight, and combinations thereof. In one embodiment the effect is selected from inducting satiety, effecting the reduction of energy intake, and combinations thereof. In yet another embodiment the effect is inducing satiety.

As used herein, the term "oral administration," "orally administering," or the like with respect to the mammal means that the mammal ingests or is directed to ingest (preferably, for the purpose of providing body weight management) one or more compositions or medicaments of the present invention. Wherein the mammal is directed to ingest one or more of the compositions, such direction may be that which instructs and / or informs the user that use of the composition may and / or will provide one or more general health and / or general physiological benefits including, but not limited to, body weight management, refreshment, and nutrition. For example, such direction may be oral direction (*e*.*g*., through oral instruction from, for example, a physician, health professional, sales professional or organization, and / or radio or television media (*i*.*e*., advertisement) or written direction (*e.g*., through written direction from, for example, a physician or other health professional (*e*.*g*., scripts), sales professional or organization (*e*.*g*., through, for example, marketing brochures, pamphlets, or other instructive paraphemalia), written media (*e*.*g*., internet, electronic mail, or other computer-related media), and / or packaging associated with the composition (*e*.*g*., a label present on a package containing the composition). As used herein, "written" means through words, pictures, symbols, and / or other visible descriptors. Such direction need not utlize the actual words used herein, for example, "body weight management", "satiety", "energy intake", "mammal", or the like, but rather use of words, pictures, symbols, and the like reasonably conveying same or similar meanings are contemplated within the scope of this invention.
The administration may be of variety of frequencies depending upon the needs or desires of treatment, for examples, daily or weekly administration of a composition described herein. Daily administration is particularly preferred, including for example once-daily, twice-daily, and / or three-times-daily administration of a composition. Once daily administration is particularly preferred.

Administration may be concomitant with ingestion of a meal, may be administered between meals, or may serve as a meal replacement. For example, the compositions of this invention may be ingested as a supplement to normal dietetic requirements. As one of ordinary skill in the art will recognize, the composition may be optimized depending upon the time or character of administration. For example, wherein the administration serves as a meal replacement, it may be advantageous to formulate the composition as a food (*e*.*g*., a meal replacement bar) or beverage. As another example, wherein the administration is concomitant with a meal or is between meals, it may be advantageous to formulate the composition as tablet, capsule, or chew.

The composition may be administered as any of a variety of forms, for example, as a food (*e*.*g*., a bar), beverage, tablet, capsule, chew, emulsion, or another orally administrable form. Wherein the form is a tablet, capsule, or the like, non-limiting techniques for preparing appropriate dosage forms are described in the following references: Modern Pharmaceutics, Chapters 9 and 10, Banker & Rhodes, eds. (1979); Lieberman et al., Pharmaceutical Dosage Forms: Tablets (1981); and Ansel, Introduction to Pharmaceutical Dosage Forms, 2nd Ed., (1976).

In one optional embodiment of the present invention, the compositions comprise an enteric delivery system, preferably a small intestinal enteric delivery system. To illustrate, for delivery of the SCFA and / or LCFA to the small intestine, the SCFA and / or LCFA may be combined with a component having a pH of about 5.5 or greater, such that the SCFA and / or LCFA bypass the stomach unabsorbed and are delivered specifically to areas of the small intestine. This may be particularly advantageous wherein the LCFA or the SCFA is the free acid (for example, not an ester of acetic, propionic, or butyric acid). Enteric delivery systems are commonly known.

Dosage will tend to vary, for example, depending upon the mammal which is administered the composition or perhaps the needs of that particular mammal. Typically, a dosage of the composition that is safe and effective will be delivered. Preferred levels of SCFA and LCFA have already been described herein. Moreover, preferred ratios of SCFA to LCFA have also been described herein. It is understood that these previously described dosage ranges are by way of example only, and that administration can be adjusted depending on various factors. For example, the specific dosage of the composition which is administered, as well as the duration of treatment, are interdependent. The dosage and treatment regimen will also depend upon such factors as the specific compound used, the treatment indication, the efficacy of the compound, the personal attributes of the mammal (such as, for example, weight, age, gender, and medical condition of the mammal), and compliance with the treatment regimen.

### EXAMPLES

The following are non-limiting examples of the present compositions, prepared utilizing conventional methods. The following examples are provided to illustrate the invention and are not intended to limit the scope thereof in any manner:

### Example 1

A citrus-flavored beverage is prepared having the following components in the indicated amounts:

| **Component** | **Weight Percent** |
|---|---|
| Sodium Citrate | 0.06 |
| Pectin | 0.3 |
| Gum Arabic | 1.5 |
| Sodium Caseinate | 0.55 |
| Calcium Caseinate | 1.18 |
| Sugar | 1.02 |
| Citric Acid | 0.28 |
| Acesulfame K | 0.01 |
| Sucralose, 25% solution | 0.04 |
| Calcium Propionate | 0.03 |
| Ethyl Oleate | 0.61 |
| Calcium Lactate | 0.04 |
| Erythritol | 1.97 |
| Flavoring Agents (including mango, peach, and vanilla flavors) | 1.55 |
| Water | *Quantum satis* |

### Example 2

A citrus-flavored beverage is prepared having the following components in the indicated amounts:

| **Component** | **Weight Percent** |
|---|---|
| Sodium Citrate | 0.06 |
| Pectin | 0.3 |
| Gum Arabic | 1.5 |
| Sodium Caseinate | 0.55 |
| Calcium Caseinate | 1.18 |
| Sugar | 1 |
| Citric Acid | 0.28 |
| Acesulfame K | 0.01 |
| Sucralose, 25% solution | 0.04 |
| Calcium Propionate | 0.03 |
| Ethyl Oleate | 0.6 |
| Emulsifier (Steroyl Lactylate, e.g., EMPLEX K, commercially Available from American Ingredients, Inc.) | 0.05 |
| Calcium Lactate | 0.04 |
| Erythritol | 1.97 |
| Flavoring Agents (including mango, peach, and vanilla flavors) | 1.55 |
| Water | *Quantum satis* |

### Example 3

A citrus-flavored beverage is prepared having the following components in the indicated amounts:

| **Component** | **Weight Percent** |
|---|---|
| Sodium Citrate | 0.06 |
| Pectin | 0.6 |
| Gum Arabic | 1.5 |
| Sodium Caseinate | 0.55 |
| Calcium Caseinate | 1.18 |
| Sugar | 1.02 |
| Citric Acid | 0.28 |
| Acesulfame K | 0.01 |
| Sucralose, 25% solution | 0.04 |
| Calcium Propionate | 0.03 |
| Ethyl Oleate | 0.61 |
| Calcium Lactate | 0.04 |
| Erythritol | 1.97 |
| Flavoring Agents (including mango, peach, and vanilla flavors) | 1.55 |
| Water | *Quantum satis* |

### Example 4

A citrus-flavored beverage is prepared having the following components in the indicated amounts:

| **Component** | **Weight Percent** |
|---|---|
| Sodium Citrate | 0.06 |
| Pectin | 0.3 |
| Gum Arabic | 1.5 |
| Sodium Caseinate | 1 |
| Calcium Caseinate | 2.3 |
| Sugar | 1.02 |
| Citric Acid | 0.28 |
| Acesulfame K | 0.01 |
| Sucralose, 25% solution | 0.04 |
| Calcium Propionate | 0.03 |
| Ethyl Oleate | 0.61 |
| Calcium Lactate | 0.04 |
| Erythritol | 1.97 |
| Flavoring Agents (including mango, peach, and vanilla flavors) | 1.55 |
| Water | *Quantum satis* |

### Example 5

A citrus-flavored beverage is prepared having the following components in the indicated amounts:

| **Component** | **Weight Percent** |
|---|---|
| Sodium Citrate | 0.06 |
| Pectin | 0.6 |
| Gum Arabic | 1.5 |
| Sodium Caseinate | 1 |
| Calcium Caseinate | 1.75 |
| Sugar | 1.02 |
| Citric Acid | 0.28 |
| Acesulfame K | 0.006 |
| Sucralose, 25% solution | 0.04 |
| Calcium Propionate | 0.03 |
| Ethyl Oleate | 0.61 |
| Calcium Lactate | 0.04 |
| Erythritol | 1.97 |
| Flavoring Agents (including mango, peach, and vanilla flavors) | 1.55 |
| Water | *Quantum satis* |

### Example 6

A composition according to any of Examples 1 - 5 is prepared according to the following process, which is performed at a substantially constant temperature of about 20 °C. Alternatively, the compositions are prepared by conventional or other means.

A Likwifier and mixing tank are provided, wherein the Likwifier is positioned to run in circulation with the mixing tank. Water is added to the Likwifier. As part of forming the first mixture, the sodium citrate, pectin, and gum arabic are added to the Likwifier in sequence under conditions of high shear for about four minutes (depending upon batch size). To finalize formation of the first mixture, the sodium caseinate, calcium caseinate, and sugar are added to the Likwifier in sequence under conditions of high shear for about four minutes, whereby the caseinates become hydrated. To form the second mixture, the citric acid is slowly added to the first mixture under conditions of high shear over a time period of about three to about thirty minutes, depending upon batch size. The acesulfame K and sucralose solution are added to the second mixture under high shear conditions for about four minutes. Circulation with the mixing tank is ceased. A pre-mixture of the calcium lactate, calcium propionate, and water is formed and added to the resulting mixture in the mixing tank under low shear conditions. The ethyl oleate (fatty acid material) is then added to the resulting mixture under low shear conditions. The remaining components are than added under low shear conditions. The composition may optionally be homogenized at about 35 MPa (5,000 psi,) sterilized under ultra-high temperature (UHT) conditions, homogenized at 24 MPa (3,500 psi,) and aseptically packed,

### Example 7

A vanilla flavored beverage composition is prepared having the following components in approximately the indicated amounts:

| **Component** | **Weight Percent** |
|---|---|
| Carrageenan (commercially available from FMC Biopolymer Food & Specialty Business, Philadelphia, PA) | 0.04 |
| Cellulose Gum (commercially available from FMC Biopolymer Food & Specialty Business, Philadelphia, PA) | 0.52 |
| Gum Arabic (commercially available from TIC Gums, Inc., Belcamp, MD) | 0.41 |
| Nutrients | 0.42 |
| Calcium Propionate | 0.03 |
| Soy Lecithin (commercially available from Central Soya, Fort | 0.03 |
| Wayne, IN) | |
| Ethyl Oleate (commercially available from Victorian Chemical Co., Richmond, Victoria Austrailia) | 1.08 |
| Sodium Caseinate | 0.3 |
| Milk Protein Concentrate (80% milk protein) (commercially available from New Zealand Milk Products, Lemoyne, PA) | 3 |
| Sugar | 4 |
| Sucralose Liquid Concentrate (commercially available from McNeil Specialty, McIntosh, AL) | 0.03 |
| Flavoring Agents, including vanilla flavor | 0.75 |
| Water | *Quantum satis* |

### Example 8

325 ml (Eleven fluid ounces) of a chocolate flavored beverage composition is prepared having the following components in approximately the indicated amounts:

| **Component** | **Weight Percent** |
|---|---|
| Sodium Hydroxide | 0.03 |
| Carrageenan (commercially available from FMC Biopolymer Food & Specialty Business, Philadelphia, PA) | 0.05 |
| Cellulose Gum (commercially available from FMC Biopolymer Food & Specialty Business, Philadelphia, PA) | 0.44 |
| Gum Arabic (commercially available from TIC Gums, Inc., Belcamp, MD) | 0.45 |
| Starch (commercially available from National Starch & Chemical, Bridgewater, NJ) | 1.5 |
| Nutrients | 0.48 |
| Calcium Propionate | 0.2 |
| Emulsifier | 0.04 |
| Ethyl Oleate (commercially available from Victorian Chemical Co., Richmond, Victoria Austrailia) | 1.18 |
| Sodium Caseinate | 0.33 |
| Milk Protein Concentrate (80% milk protein) (commercially available from New Zealand Milk Products, Lemoyne, PA) | 3.23 |
| sugar | 4 |
| Maltodextrin | 2 |
| Sucralose Liquid Concentrate (commercially available from McNeil Specialty, McIntosh, AL) | 0.06 |
| Flavoring Agents, including cocoa | 2.75 |
| Water | *Quantum satis* |

### Example 9

325 ml (Eleven fluid ounces) of a chocolate flavored beverage composition is prepared having the following components in approximately the indicated amounts:

| **Component** | **Weight Percent** |
|---|---|
| Sodium Polyphosphate | 0.01 |
| Sodium Hydroxide | 0.03 |
| Carrageenan (commercially available from FMC Biopolymer Food & Specialty Business, Philadelphia, PA) | 0.1 |
| Cellulose Gum (commercially available from FMC Biopolymer Food & Specialty Business, Philadelphia, PA) | 0.44 |
| Gum Arabic (commercially available from TIC Gums, Inc., Belcamp, MD) | 2.17 |
| Nutrients, including Calcium Propionate | 0.74 |
| Emulsifier | 0.04 |
| Ethyl Oleate (commercially available from Victorian Chemical Co., Richmond, Victoria Austrailia) | 1.18 |
| Flavoring Agents, including cocoa | 1.75 |
| Silicone Emulsion | 0.002 |
| Sodium Caseinate | 0.33 |
| Milk Protein Concentrate (80% milk protein) (commercially available from New Zealand Milk Products, Lemoyne, PA) | 4.7 |
| White Sugar | 4 |
| Maltodextrin | 3 |
| Sucralose Liquid Concentrate (commercially available from McNeil Specialty, McIntosh, AL) | 0.06 |
| Water | *Quantum satis* |

### Example 10

325 ml (Eleven fluid ounces) of a chocolate flavored beverage composition is prepared having the following components in approximately the indicated amounts:

| **Component** | **Weight Percent** |
|---|---|
| Carrageenan (commercially available from FMC Biopolymer Food & Specialty Business, Philadelphia, PA) | 0.06 |
| Cellulose Gum (commercially available from FMC Biopolymer Food & Specialty Business, Philadelphia, PA) | 0.49 |
| Gum Arabic (commercially available from TIC Gums, Inc., Belcamp, MD) | 0.45 |
| Nutrients, including calcium propionate | 0.65 |
| Emulsifier | 0.04 |
| Ethyl Oleate (commercially available from Victorian Chemical Co., Richmond, Victoria Austrailia) | 1.18 |
| Sodium Caseinate | 0.33 |
| Milk Protein Concentrate (80% milk protein) (commercially available from New Zealand Milk Products, Lemoyne, PA) | 1.43 |
| Nonfat Dry Milk Powder (commercially available from O-AT-KA Milk Products Cooperative, Inc., Batavia, KY) | 4.23 |
| White Sugar | 4 |
| Maltodextrin | 3 |
| Sucralose Liquid Concentrate (commercially available from McNeil Specialty, McIntosh, AL) | 0.06 |
| Flavoring Agents, including cocoa | 1.7 |
| Water | *Quantum satis* |

### Example 11

325 ml (Eleven fluid ounces) of a chocolate flavored beverage composition is prepared having the following components in approximately the indicated amounts:

| **Component** | **Weight Percent** |
|---|---|
| Sodium Polyphosphate | 0.01 |
| Sodium Hydroxide | 0.03 |
| Carrageenan (commercially available from FMC Biopolymer Food & Specialty Business, Philadelpia, PA) | 0.06 |
| Cellulose Gum (commercially available from FMC Biopolymer Food & Specialty Business, Philadelphia, PA) | 0.44 |
| Gum Arabic (commercially available from TIC Gums, Inc., Belcamp, MD) | 0.45 |
| Nutrients, including calcium propionate | 0.65 |
| Emulsifier | 0.04 |
| Ethyl Oleate (commercially available from Victorian Chemical Co., Richmond, Victoria Austrailia) | 1.18 |
| Sodium Caseinate | 0.33 |
| Milk Protein Concentrate (80% milk protein) (commercially available from New Zealand Milk Products, Lemoyne, PA) | 2.31 |
| Nonfat Dry Milk Powder (commercially available from O-AT-KA Milk Products Cooperative, Inc., Batavia, KY) | 2.14 |
| White Sugar | 4 |
| Maltodextrin | 3 |
| Sucralose Liquid Concentrate (commercially available from McNeil Specialty, McIntosh, AL) | 0.06 |
| Flavoring Agents, including cocoa | 1.7 |
| Water | *Quantum satis* |

### Example 12

A composition according to any of Examples 7 - 11 is prepared according to the following process, Alternatively, the compositions are prepared by conventional or other means.
Water is added to a high shear mixing apparatus and agitation is commenced (optionally, the apparatus may be in recirculation with, for example, a mixing tank to enable preparation of large batch sizes). The agitation may be a mixing energy of, for example, from about 5 Watt/Kg (watts per kilogram) to about 150 W/kg. A specific order of addition of the various components of the composition may not be critical to achieve the stabilizing and flavor masking benefits of the present compositions. After the addition of carrageenans or other like materials (if used), the batch is allowed to mix for about 1 to 10 minutes to allow proper hydration. Otherwise, all components may be added sequentially with little or no waiting period between the addition of each component. Once the various components are added, the composition may optionally be homogenized at a pressure of from about 23 MPa (3,000 psi) to about 49 MPa (7,000 psi.) The composition may optionally be sterilized under ultra-high temperature (UHT) conditions. The composition may optionally be homogenized at a pressure of from about 14 MPa (2,000 psi) to about 35 MPa (5,000 psi.) Typically, homogenization occurs either prior or subsequent to sterilization, but homogenization may occur both prior and subsequent to homogenization. The composition may be optionally aseptically filled into an appropriate aseptic containing device.
The means for subjecting the components to the mixing energy may be selected from a variety of well-known apparatuses (energizing means). For example, this energizing means may be a mixer which provides energy to the liquid medium by forming ultrasonic vibrations therein, *e*.*g*., a Sonolator, commercially available from Sonic Corporation, Stratford, CT or Piezoelectric transducers. The Sonolator is an in-line system providing ultrasonic vibrations by pumping a liquid, a blend of liquids, or a solid dispersion in a liquid through a shaped orifice at a high linear velocity. The liquid stream impinges against a blade cantilevered in the stream. Flow over the blade causes vibrations in the blade that produces cavitation in the stream converting flow energy into mixing/dispersion energy. Other particularly useful energizing means include batch mixers providing a high agitator tip speed, e.g., blenders as available from Sunbeam Corporation of Delray Beach, FL with the brand name OSTERIZER. Additionally rotor/stator high shear mixers, commercially available from Charles Ross & Son, Hauppauge, NY may be useful. In-line mixers such as are available from Quadro Inc., Millburn, NJ, as model Quadro ZC/XC are useful as well. Additionally, particularly preferred energizing means for use herein include bottom-driven mixtures such as the Breddo Likwifier (Model LOR, round tank; Model LTD square tank) commercially available from Breddo Likwifier, Kansas City, MO and APV Mixer/Blender (Multiverter (round tank) / Liquiverter (square tank) high speed mixers commercially available from APV Crepaco, Inc., Lake Mills, WI.

### Example 13

A supplement health bar is prepared having the following components in approximately the indicated amounts. The bar is prepared using conventional methods.

| **Component** | **Weight Percent** |
|---|---|
| Ethyl Oleate | 5.6 |
| High Fructose Com Syrup | 15 |
| Granola | 16.4 |
| Oat Fiber | 3.2 |
| Honey | 4.8 |
| Soy | 16.9 |
| Wheat Bran | 4.7 |
| Bran Flakes | 3.8 |
| Chocolate | 12 |
| Calcium Propionate | 0.3 |
| Carbohydrate Sugars | 12.8 |
| Flavors | 0.7 |
| Glycerin | 3 |
| Salt | 0.2 |
| Calcium Carbonate | 0.6 |

### Example 14

A meal replacement health bar is prepared having the following components in approximately the indicated amounts. The bar is prepared using conventional methods.

| **Component** | **Weight Percent** |
|---|---|
| Ethyl Oleate | 6.6 |
| High Fructose Corn Syrup | 15.5 |
| Granola | 15.8 |
| Oat Fiber | 3.35 |
| Honey | 6.13 |
| Soy | 21 |
| Wheat Bran | 4.1 |
| Bran Flakes | 8 |
| Nutrients | 3.1 |
| Calcium Propionate | 0.17 |
| Carbohydrate Sugars | 12.7 |
| Flavors | 0.35 |
| Glycerin | 3 |
| Salt | 0.2 |

### Example 15

A capsule composition is prepared, wherein the capsule contains the following components in approximately the indicated amounts. The capsule is prepared using conventional methods.

| **Component** | **Weight Percent** |
|---|---|
| Ethyl Oleate | 79% |
| Calcium Propionate | 21% |

The components are encapsulated in a size "00" capsule, commercially available from a variety of sources. A capsule containing about 1.5 grams of the ethyl oleate and about 0.4 grams of the calcium propionate is dosed daily to a female human consumer weighing about 70 kilograms. The consumer experiences a weight loss of about 1Kg (2 pounds) per week for a period of 4 weeks.

## Claims

1. A composition, for oral administration to a mammal, comprising:
a) from 0.001% to 8% of a short chain fatty acid component selected from acetic acid, propionic acid and butyric acid; methyl, ethyl, n-propyl, iso-propyl, n-butyl and isobutyl esters thereof; salts thereof; and mixtures thereof; and
b) from 0.001% to 10% of a long chain fatty acid component selected from C₁₀ - C₂₄ fatty acids, non-glyceryl esters of C₁₀ - C₂₄ fatty acids, and mixtures thereof.

2. The composition according to Claim 1 wherein the ratio of long chain fatty acid component to short chain fatty acid component is 10:1 or less, by weight.

3. The composition according to any of the preceding claims wherein the short chain fatty acid component is selected from propionic acid, salts of propionic acid, and mixtures thereof.

4. The composition according to any of the preceding claims wherein the short chain fatty acid component is selected from calcium propionate, sodium propionate, magnesium propionate, potassium propionate, and mixtures thereof and wherein the long chain fatty acid component is selected from oleic acid, linoleic acid, methyl oleate, ethyl oleate, *n-*propyl oleate, *iso*-propyl oleate, *n*-butyl oleate, *iso*-butyl oleate, methyl linoleate, ethyl linoleate, *n*-propyl linoleate, *iso*-propyl linoleate, *n*-butyl linoleate, *iso*-butyl linoleate, and mixtures thereof.

5. The composition according to any of the preceding claims wherein the short chain fatty acid component is calcium propionate and the long chain fatty acid component is selected from oleic acid, linoleic acid, ethyl oleate, and mixtures thereof.

6. The composition according to any of the preceding claims further comprising from 0.1% to 10% fiber.

7. The composition according to any of the preceding claims which is a beverage composition comprising at least 70% water.

8. The use of a short chain fatty acid component selected from acetic acid, propionic acid and butyric acid; methyl, ethyl, n-propyl, iso-propyl, n-butyl and isobutyl esters thereof; salts thereof; and mixtures thereof; in combination with a long chain fatty acid component selected from C₁₀ - C₂₄ fatty acids, non-glyceryl esters of C₁₀ - C₂₄ fatty acids, and mixtures thereof, in the manufacture of a medicament for managing the body weight of a mammal.

9. The use according to Claim 8 wherein the short chain fatty acid component is calcium propionate and the long chain fatty acid component is selected from oleic acid, linoleic acid, ethyl oleate, and mixtures thereof.

10. The use according to Claim 8 or Claim 9 wherein the medicament is a beverage composition comprising at least 70% water.

## Patentansprüche

1. Zusammensetzung zur oralen Verabreichung an ein Säugetier, umfassend:
(a) von 0,001 % bis 8 % einen kurzkettigen Fettsäurebestandteil, der ausgewählt ist aus Essigsäure, Propionsäure und Buttersäure; Methyl-, Ethyl-, n-Propyl-, Isopropyl-, n-Butyl- und Isobutylestem davon; Salzen davon; und Mischungen davon; und
(b) von 0,001 % bis 10 % einen langkettigen Fettsäurebestandteil, der ausgewählt ist aus C₁₀ - C₂₄-Fettsäuren, Nicht-Glycerylestern von C₁₀-C₂₄-Fettsäuren und Mischungen davon;

2. Zusammensetzung nach Anspruch 1, wobei das Verhältnis des langkettigen Fettsäurebestandteils zu dem kurzkettigen Fettsäurebestandteil bezogen auf das Gewicht 10:1 oder weniger beträgt.

3. Zusammensetzung nach einem der vorstehenden Ansprüche, wobei der kurzkettige Fettsäurebestandteil ausgewählt ist aus Propionsäure, Salzen von Propionsäure und Mischungen davon.

4. Zusammensetzung nach einem der vorstehenden Ansprüche, wobei der kurzkettige Fettsäurebestandteil ausgewählt ist aus Calciumpropionat, Natriumpropionat, Magnesiumpropionat, Kaliumpropionat und Mischungen davon, und wobei der langkettige Fettsäurebestandteil ausgewählt ist aus Ölsäure, Linolsäure, Methyloleat, Ethyloleat, n-Propyloleat, Isopropyloleat, n-Butyloleat, Isobutyloleat, Methyllinoleat, Ethyllinoleat, n-Propyllinoleat, Isopropyllinoleat, n-Butyllinoleat, Isobutyllinoleat und Mischungen davon.

5. Zusammensetzung nach einem der vorstehenden Ansprüche, wobei der kurzkettige Fettsäurebestandteil Calciumpropionat ist und der langkettige Fettsäurebestandteil ausgewählt ist aus Ölsäure, Linolsäure, Ethyloleat und Mischungen davon.

6. Zusammensetzung nach einem der vorstehenden Ansprüche, ferner umfassend von 0,1 % bis 10 % Faser.

7. Zusammensetzung nach einem der vorstehenden Ansprüche, die eine Getränkezusammensetzung ist, die zu mindestens 70 % Wasser umfasst.

8. Verwendung eines kurzkettigen Fettsäurebestandteils, der ausgewählt ist aus Essigsäure, Propionsäure und Buttersäure; Methyl-, Ethyl-, n-Propyl-, Isopropyl-, n-Butyl- und Isobutylestern davon; Salzen davon; und Mischungen davon; in Kombination mit einem langkettigen Fettsäurebestandteil, der ausgewählt ist aus C₁₀ - C₂₄-Fettsäuren, Nicht-Glycerylestern von C₁₀ - C₂₄-Fettsäuren und Mischungen davon, bei der Herstellung eines Medikaments zur Steuerung des Körpergewichts eines Säugetiers.

9. Zusammensetzung nach Anspruch 8, wobei der kurzkettige Fettsäurebestandteil Calciumpropionat ist und der langkettige Fettsäurebestandteil ausgewählt ist aus Ölsäure, Linolsäure, Ethyloleat und Mischungen davon.

10. Zusammensetzung nach Anspruch 8 oder 9, wobei das Medikament eine Getränkezusammensetzung ist, die zu mindestens 70 % Wasser umfasst.

## Revendications

1. Composition, pour administration orale à un mammifère, comprenant :
(a) de 0,001 % à 8 % d'un composant acide gras à courte chaîne choisi parmi l'acide acétique, l'acide propionique et l'acide butyrique ; leurs esters de méthyle, éthyle, n-propyle, n-butyle et isobutyle ; leurs sels, et leurs mélanges ; et
(b) de 0,001 % à 10 % d'un composant acide gras à longue chaîne choisi parmi des acides gras en C₁₀ à C₂₄, des esters non glycériques d'acides gras en C₁₀ à C₂₄, et leurs mélanges.

2. Composition selon la revendication 1, dans laquelle le rapport de composant acide gras à longue chaîne sur composant acide gras à courte chaîne est 10 : 1 ou moins, en poids.

3. Composition selon l'une quelconque des revendications précédentes, dans laquelle le composant acide gras à courte chaîne est choisi dans le groupe constitué d'acide propionique, sels d'acide propionique, et leurs mélanges.

4. Composition selon l'une quelconque des revendications précédentes, dans laquelle le composant acide gras à courte chaîne est choisi dans le groupe constitué de propionate de calcium, propionate de sodium, propionate de magnésium, propionate de potassium, et leurs mélanges et dans laquelle le composant acide gras à longue chaîne est choisi dans le groupe constitué d'acide oléique, acide linoléique, oléate de méthyle, oléate d'éthyle, oléate de n-propyle, oléate d'iso-propyle, oléate de n-butyle, oléate d'iso-butyle, linoléate de méthyle, linoléate d'éthyle, linoléate de n-propyle, linoléate d'iso-propyle, linoléate de n-butyle, linoléate d'iso-butyle, et leurs mélanges.

5. Composition selon l'une quelconque des revendications précédentes, dans laquelle le composant acide gras à courte chaîne est du propionate de calcium, et le composant acide gras à longue chaîne est choisi parmi l'acide oléique, l'acide linoléique, l'oléate d'éthyle, et leurs mélanges.

6. Composition selon l'une quelconque des revendications précédentes, comprenant, en outre, de 0,1 % à 10 % de fibre.

7. Composition selon l'une quelconque des revendications précédentes qui est une composition de boisson comprenant au moins 70 % d'eau

8. Utilisation d'un composant acide gras à courte chaîne choisi parmi l'acide acétique, l'acide propionique, l'acide butyrique ; leurs esters de méthyle, éthyle, n-propyle, n-butyle et isobutyle ; leurs sels ; et leurs mélanges : en combinaison avec un composant acide gras à longue chaîne choisi parmi les acides gras en C₁₀ à C₂₄, les esters non glycériques d'acides gras en C₁₀ à C₂₄, et leurs mélanges, dans la fabrication d'un médicament pour prendre en charge le poids corporel d'un mammifère.

9. Composition selon la revendication 8, dans laquelle le composant acide gras à courte chaîne est du propionate de calcium et le composant acide gras à longue chaîne est choisi dans le groupe constitué de l'acide oléique, l'acide linoléique, l'oléate d'éthyle, et leurs mélanges.

10. Composition selon la revendication 8 ou la revendication 9, dans laquelle le médicament est une composition de boisson comprenant au moins 70 % d'eau.
